# EUROPEAN PATENT APPLICATION

(11) **EP 1 350 781 A1**
(43) Date of publication of application: **08.10.2003**
(21) Application number: 01273182.4
(22) Date of filing: 17.12.2001
(51) Int. Cl.: C07C 37/20, C07C 39/16

(54) **PROCESS FOR PRODUCING BISPHENOL A**

(30) Priority: 11.01.2001 JP 2001003632
(71) Applicant: IDEMITSU PETROCHEMICAL CO., LTD., Tokyo 130-0015 (JP)
(72) Inventor: SARUWATARI, Tetsuya, Tokuyama-shi, Yamaguchi 745-0843 (JP)
(74) Representative: Gille Hrabal Struck Neidlein Prop Roos
(86) International application number: JP0111036
(87) International publication number: WO02055462

(57) **Abstract**

In the production of bisphenol A by condensation of phenol and acetone with the use of a cation exchange resin as a catalyst and a free mercaptan as a promoter, the superficial velocity of raw material within the reaction column packed with a cation exchange resin is controlled to be in the range of 1.5m/hr to 6m/hr.

With this method, the degree of conversion of phenol can be improved, and bisphenol A can be effectively produced.

## Description

### TECHNICAL FIELD

The present invention relates to a method of producing bisphenol A. More specifically, the present invention relates to a method of producing bisphenol A in which, in the production of bisphenol A [2,2-bis (4-hydroxyphenyl) propane] from phenol and acetone with the use of a cation exchange resin as a catalyst and a free mercaptan as a promoter, the degree of conversion of phenol is improved by controlling the supertcial velocity of raw material within a reaction column, so that bisphenol A is effectively produced.

### BACKGROUND OF THE INVENTION

Bisphenol A has been known as an important compound for raw material for engineering plastics, such as polycarbonate resins, polyacrylate resins, etc, or for epoxy resins, and the demand for it tends to be still more growing recently.

Bisphenol A is produced by the condensation of an excess of phenol and acetone in the presence of an acid catalyst and optionally a promoter, such as a sulfur compound, etc.

As the acid catalyst for that reaction, inorganic mineral acids, such as sulfuric acid, hydrochloric acid, etc. were conventionally used. However, cation exchange resins have recently attracted attention (GB Patent Nos. 842209, 849565 and 883391), and have come to be industrially used.

On the other hand, it has been known that as for sulfur compounds used as the promoter, alkyl mercaptans with or without substituting groups, such as methyl mercaptan, ethyl mercaptan, thioglycolic acid, etc., are useful (U.S. Patent Nos. 2,359,242 and 2,775,620). The mercaptans function to increase the reaction rate and improve the selectivity. For example, as reaction by-products in the production of bisphenol A, 2-(2-hydroxyphenyl)-2-(4-hydroxyphenyl) propane (a combination of o- and p'- types) is mainly formed, and tris-phenol, polyphenol, etc. are also formed. Especially, in cases where bisphenol A is used as raw material for polycarbonate resins, polyacrylate resins, etc., required is colorless high purity bisphenol A containing a reduced amount of those by-products. To this end, mercaptans are used as a promoter in order not only to increase the reaction rate but also to suppress the formation of the by-products and increase the selectivity.

In cases where bisphenol A is industrially produced by condensation of phenol and acetone, a method is generally used in which phenol and acetone as raw material and a mercaptan as a promoter are continuously fed to a reaction column packed with the above-mentioned cation exchange resin.

With respect to the flow within the reaction column in this way of reaction, it is taught, for example, that (1) in cases where the direction of flow is downward, pressure loss is generated at the ion exchange resin bed, resulting in the reduction in the yield of bisphenol A (Japanese Unexamined Patent Publication No. 6(1994)-320009), and (2) in order to have the material mixture liquid flow within the reaction column at a high flow rate, it is required to feed the material mixture liquid at a pressure extremely high relative to that of the reaction column, and this tendency is greater in the case where gel type resins are used as the ion exchange resin (Japanese Unexamined Patent Publication No. 6(1994)-340563).

As is mentioned above, only the pressure loss in the reaction column has been recognized as a problem in the conventional art, and it is the fact that there are heretofore no findings as to what flow rate for the raw material is adequate in terms of the degree of conversion and the deterioration of the catalyst.

### DISCLOSURE OF THE INVENTION

An object of the present invention is to provide an industrially useful method of producing bisphenol A in which, in the production of bisphenol A from phenol and acetone with the use of a cation exchange resin as a catalyst and a mercaptan as a promoter, the degree of conversion of phenol is improved by controlling the flow rate of the raw material within a reaction column, so that bisphenolAis effectively produced.

The inventors of the present invention have found, through extensive studies to achieve the above-mentioned object, that the degree of conversion of phenol is reduced if the superficial velocity of raw material is less than a certain value and on the other hand the rate of deterioration of the catalyst becomes higher and greater increase in the pressure loss also results as the superficial velocity of raw material increases, and that in a certain range of the superficial velocity of raw material high degree of conversion of phenol is obtained and the pressure loss is not very large. The present invention has been made based on the above finding.

Specifically, the present invention provides a method of producing bisphenol A, in which, in the production of bisphenol A by condensation of phenol and acetone with the use of a cation exchange resin as a catalyst and a free mercaptan as a promoter, the superficial velocity of raw material within a reaction column packed with a cation exchange resin is controlled to be in the range of 1.5m/hr to 6m/hr.

### BEST MODE FOR CARRYING OUT THE INVENTION

The method according to the present invention is a method of producing bisphenol A in which phenol and acetone are condensed with the use of a cation exchange resin as a catalyst and a free mercaptan as a promoter. There is no specific limitation with respect to the kind of the cation exchange resin to be used, and any of those which are conventionally employed as catalysts for the production of bisphenol A can be used. However, sulfonic acid type cation exchange resins are preferred especially in terms of the catalytic activity.

There is no specific limitation with respect to the kind of the sulfonic acid type cation exchange resins to be used inasmuch as they are strong acidic cation exchange resins having sulfonic groups. Examples of the sulfonic acid type cation exchange resin include sulfonated styrene-divinyl benzene copolymer, sulfonated cross-linked styrene polymer, phenol formaldehyde-sulfonic acid resin, benzene formaldehyde-sulfonic acid resin, etc. These may be used singly or in combination.

On the other hand, the free mercaptan as the promoter as used herein means a compound having a free form of SH group in the molecule. As the free mercaptan, an alkyl mercaptan can be adopted, which may be either of a non-substituted alkyl mercaptan and a substituted alkyl mercaptan having at least one substituting group, such as a carboxylic group, an amino group, a hydroxyl group, etc. Examples of non-substituted alkyl mercaptan include methyl mercaptan, ethyl mercaptan, n-butyl mercaptan, n-octyl mercaptan, etc. Examples of the substituted alkyl mercaptan include mercaptocarboxylic acids such as thioglycolic acid, β - mercaptopropionic acid, etc., aminoalkane thiols such as 2-amino ethane thiol, 2,2-dimethyl thiazolidine, etc., mercaptoalcohols, such as mercaptoethanol, etc. Among these, the non-substituted alkyl mercaptans are especially preferred in terms of the promoting action. In addition, these mercaptans may be used singly or in combination.

The amount of each of these mercaptans is generally selected to be in the range of 0.1-20 mole %, preferably in the range of 1-10 mole %, relative to acetone, which is one of the raw materials to be used.

Further, there is no specific limitation with respect to the ratio of the amount between phenol and acetone, but it is desirable that the amount of unreacted acetone is as small as possible in terms of the easiness of purification of the produced bisphenol A and from an economical point of view. Therefore, it is advantageous that phenol is employed in an amount in excess of its stoichiometric amount. Generally, phenol is employed in an amount of 3-30 moles, preferably 5-15 moles, per one mole of acetone.

Meanwhile, the method of producing bisphenol A according to the present invention does not generally require a reaction solvent except for the cases where the reaction is carried out at such low temperatures that the viscosity of the reaction liquid is too high or the reaction liquid solidifies resulting in difficulty in operation.

To effect the condensation reaction of phenol and acetone in the present invention, there can be used a fixed bed continuous reaction system in which phenol, acetone and the above-explained free mercaptan are continuously fed to a reaction column packed with the cation exchange resin as an acid catalyst and are reacted. In this respect, the reaction can be carried out with one reaction column, but two or more reaction columns may be used so that they are arranged in series or in parallel. It is industrially particularly advantageous to arrange two or more reaction columns each packed with the cation exchange resin in series and to use a fixed bed multiple stage continuous reaction system.

The reaction conditions for the fixed bed continuous reaction system will hereinbelow be explained.

The molar ratio of acetone/phenol in this reaction is generally selected to be in the range of 1/30 to 1/3, and preferably in the range of 1/15 to 1/5. If this molar ratio is lower than 1/30, there is a risk that the reaction rate becomes too low. If the molar ratio is greater than 1/3, more impurities are generated and the selectivity of bisphenol A tends to be lower.

Meanwhile, the molar ratio of the free mercaptan/acetone is generally selected to be in the range of 0.1/100 to 20/100, and preferably in the range of 1/100 to 10/100. If this molar ratio is lower than 0.1/100, there is a risk that improvements with respect to the reaction rate and the selectivity of bisphenol A are not sufficiently obtained. If this molar ratio is greater than 20/100, advantages are not fully enjoyed relative to the amount of the free mercaptan used.

The reaction temperature is generally selected to be in the range of 40-150°C, and preferably in the range of 60-110°C. If the reaction temperature is lower than 40°C, the reaction rate becomes low and the viscosity of the reaction liquid becomes extremely high which may create a risk of solidification. If the reaction temperature exceeds 150°C, it becomes difficult to control the reaction, the selectivity of bisphenol A (a combination of p- and p'- types) is lowered, and the cation exchange resin as a catalyst may decompose or deteriorate.

In the method according to the present invention, the flow rate of the raw material within the reaction column is controlled in order to enhance the degree of conversion of phenol. Specifically, if the superficial velocity of raw material within the reaction column is less than 1.5m/hr, the degree of conversion of phenol is lowered. On the other hand, the rate of deterioration of the cation exchange resin increases and the pressure loss rises as the superficial velocity of raw material is increased. Therefore, in order to achieve high degrees of conversion, the superficial velocity of raw material should be controlled to be in the range of 1.5m/hr to 6m/hr. If the superficial velocity of raw material is within the above range, the pressure loss does not become so high. LHSV (Liquid Hourly Space Velocity) of the material mixture is generally selected to be in the range of 0.2 hr⁻¹ to 50 hr⁻¹, and preferably in the range of 0.5 hr⁻¹ to 30 hr⁻¹.

In addition, in the method according to the present invention, it is desirable, in terms of the degree of conversion of phenol, that the reaction column has a ratio of L (height)/D (diameter) of 1 or less.

In the method according to the present invention, the reaction mixture coming from the reaction column is subjected to a post treatment in a conventional way, whereby bisphenol A is obtained.

Explaining an example of the post treatment, concentration is first carried out prior to crystallization. Although there is no specific limitation with respect to the conditions under which the concentration is carried out, the concentration is generally carried out under the conditions in which the temperature is in the range of 130°C to 170°C and the pressure is in the range of 13kPa to 53kPa. If the temperature is lower than 130°C, high vacuum is requires. If the temperature is higher than 170°C, more impurities are generated and coloring is caused thereby. Further, it is advantageous that the concentration of bisphenol A in the concentrated residue ranges from 25 wt.% to 40 wt.%. If this concentration is less than 25 wt.%, the yield of bisphenol A is low. If this concentration exceed 40 wt.%, it becomes difficult to carry the slurry after the crystallization.

Crystallization of an addition product composed of bisphenol A and phenol from the concentrated residue is generally carried out by means of the vacuum cooling crystallization method in which cooling is performed using evaporation latent heat of water under reduced pressure. In the vacuum cooling crystallization method, water is added to the concentrated residue in an amount of 3-20 wt.%, and the crystallization treatment is carried out generally at a temperature of 40-70°C and a pressure of 3-13kPa. If the amount of water added is less than 3 wt.%, heat removing capability is insufficient, and if this amount exceeds 20 wt.%, dissolution loss of bisphenol A becomes large, both of which cases are not desirable. Further, if the temperature of the crystallization treatment is lower than 40°C, there is a risk of increase in the viscosity after the crystallization and occurrence of solidification. If the temperature of the crystallization treatment exceeds 70°C, dissolution loss of bisphenol A becomes large. Both of these cases are not desirable.

Thereafter, the addition product composed of bisphenol A and phenol as thus obtained by way of the crystallization treatment is separated by a conventional method, and is then subjected to a washing treatment generally using phenol. After that, the washed addition product is subjected to a separation processing into bisphenol A and phenol. The temperature at which the separation processing is carried out is generally selected to be in the range of 130-200°C, and preferably in the range of 150-180°C. The pressure at which the separation processing is carried out is generally selected to be in the range of 3-20kPa.

High quality bisphenol A can be obtained from the bisphenol A thus obtained from the separation processing through removing the residual phenol in the latter bisphenol A substantially completely by the steam striping method, etc.

### (Examples)

The present invention will hereinbelow be described in further detail based on examples. However, the present invention is not limited to such examples in any way.

### Example 1:

A cylindrical vessel having an inner diameter of 10 mm and a length of 1500 mm was packed with a cation exchange resin (sulfonated styrene-divinyl benzene copolymer available from Mitsubishi Chemical Corporation; Product Name: DIAION SK 104) in an amount of 14 milliliters. Then, phenol at a flow rate of 300 g/hr, acetone at a flow rate of 25 g/hr and ethyl mercaptan at a flow rate of 1.3 g/hr were continuously fed to this reaction column, and were allowed to react at 75°C.

In this case, the molar ratio of acetone/phenol was set to be 1/10, LHSV (Liquid Hourly Space Velocity) was set to be 30 hr⁻¹, and the superficial velocity of raw material was set to be 2 m/hr.

After 48 hours run, the average degree of conversion of phenol was 6 % and the specific activity was 0.6. In this respect, the term, relative activity as used herein means the degree of conversion of phenol/the initial degree of conversion of phenol.

### Example 2:

The reaction was carried out in the same manner as in Example 1 except that the superficial velocity of raw material was changed to 3.8m/hr while the reaction temperature was maintained to be 75°C, the molar ratio of acetone/phenol was maintained to be 1/10, and LHSV was maintained to be 30 hr⁻¹. In this respect, the amount of the cation exchange resin was adjusted so as to have LHSV maintained to be 30 hr⁻¹.

After 48 hours run, the average degree of conversion of phenol was 6 % and the specific activity was 0.6.

### Comparative Example 1:

The reaction was carried out in the same manner as in Example 2 except that the superficial velocity of raw material was changed from 3.8m/hr to 0.5m/hr.

After 48 hours run, the average degree of conversion of phenol was 4 % and the specific activity was 0.6.

Comparing Comparative Example 1 with Example 2, it is recognized that the overall degree of conversion was lowered although the rate of deterioration was the same.

### Comparative Example 2:

The reaction was carried out in the same manner as in Example 2 except that the superficial velocity of raw material was changed from 3.8m/hr to 8m/hr.

After 48 hours run, the average degree of conversion of phenol was 4.5 % and the specific activity was 0.4.

Comparing Comparative Example 2 with Example 2, it is recognized that the average degree of conversion was lowered due to the increase in the rate of deterioration although the initial degree of conversion of phenol was not changed.

### INDUSTRIAL APPLICABILITY

According to the present invention, in the production of bisphenol A from phenol and acetone with the use of a cation exchange resin as a catalyst and a free mercaptan as a promoter, the degree of conversion of phenol is improved by controlling the superficial velocity of raw material within the reaction column, so that bisphenol A can be effectively produced.

## Claims

1. A method of producing bisphenol A, in which, in the production of bisphenol A by condensation of phenol and acetone with the use of a cation exchange resin as a catalyst and a free mercaptan as a promoter, the superficial velocity of raw material within a reaction column packed with a cation exchange resin is controlled to be in the range of 1.5m/hr to 6m/hr.

2. A method of producing bisphenol A according to Claim 1, wherein the reaction column has a ratio of L (height)/D (diameter) of 1 or less.

3. A method of producing bisphenol A according to Claim 1, wherein the cation exchange resin is a sulfonic acid type cation exchange resin.

4. A method of producing bisphenol A according to Claim 1, wherein the free mercaptan is an alkyl mercaptan, mercaptocarboxylic acid, aminoalkane thiol or mercaptoalcohol.

5. A method of producing bisphenol A according to Claim 1, wherein the molar ratio of acetone/phenol is in the range of 1/30-1/3.

6. A method of producing bisphenol A according to Claim 1, wherein the molar ratio of the free mercaptan/acetone is in the range of 0.1/100-20/100.

7. A method of producing bisphenol A according to Claim 1, wherein the condensation reaction is carried out at a temperature in the range of 40-150°C.
